# EUROPEAN PATENT SPECIFICATION

(11) **EP 3 420 362 B1**
(45) Date of publication and mention of the grant of the patent: **22.03.2023**
(21) Application number: 17708323.5
(22) Date of filing: 24.02.2017
(51) Int. Cl.: G01N 33/68, C07K 16/00

(54) **ANTIBODIES**
ANTIKÖRPER
ANTICORPS

(30) Priority: 25.02.2016 GB 201603291; 29.07.2016 GB 201613158
(43) Date of publication of application: 02.01.2019
(73) Proprietor: The Binding Site Group Limited, Birmingham, West Midlands B15 1QT (GB)
(72) Inventor: WALLIS, Gregg, Birmingham West Midlands B15 1QT (GB); ASHBY, Jamie, Birmingham West Midlands B15 1QT (GB); HARDING, Stephen, Birmingham West Midlands B15 1QT (GB)
(74) Representative: Elsy, David
(86) International application number: PCT/GB2017/050492
(87) International publication number: WO 2017/144903

(56) References cited:
- WO-A1-2014/084607
- WO-A2-2006/099481
- KR-A- 20090 094 822
- DANIEL A. RICHARDS ET AL: "Photochemically re-bridging disulfide bonds and the discovery of a thiomaleimide mediated photodecarboxylation of C-terminal cysteines", ORGANIC & BIOMOLECULAR CHEMISTRY, vol. 14, no. 2, 25 November 2015 (2015-11-25), pages 455-459, XP055367263, GB ISSN: 1477-0520, DOI: 10.1039/C5OB02120K
- GOLDBERG M ET AL: "Specific interchain cross-linking of antibodies using bismaleimides. Repression of ligand leakage in immunoaffinity chromatography", MEDLINE, 1991, XP55367595,

## Description

Antibody molecules (also known as immunoglobulins) have a twofold symmetry and typically are composed of two identical heavy chains and two identical light chains, each containing variable and constant domains. The variable domains of the heavy and light chains combine to form an antigen-binding site, so that both chains contribute to the antigen-binding specificity of the antibody molecule. The basic tetrameric structure of antibodies comprises two heavy chains covalently linked by a disulphide bond. Each heavy chain is in turn attached to a light chain, again via a disulphide bond. This produces a substantially "Y"-shaped molecule.

Heavy chains are the larger of the two types of chain found in antibodies, with typical molecular mass of 50,000-77,000 Da, compared with the smaller light chain (25,000 Da).

There are five main classes or class or classes of heavy chain which are γ, α, µ, δ and ε which are the constituents heavy chains for: IgG, IgA, IgM, IgD and IgE respectively. IgG is the major immunoglobulin of normal human serum, accounting for 70-75% of the total immunoglobulin pool. This is the major antibody of secondary immune responses. It forms a single tetramer of two heavy chains plus two light chains.

IgM accounts for approximately 10% of the immunoglobulin pool. The molecules, together with J-chains, form a pentamer of five of the basic 4-chain structures. The individual heavy chains have a molecular weight of approximately 65,000 Da and the whole molecule has a molecular weight of about 970,000 Da. IgM is largely confined to the intravascular pool and is the predominant early antibody.

IgA represents 15-20% of human serum immunoglobulin pool. More than 80% of IgA occurs as a monomer. However, some of the IgA (secretory IgA) exists as a dimeric form.

IgD accounts for less than 1% of the total plasma immunoglobulin.

IgE, although scarce in normal serum, is found on the surface membrane of basophils and mast-cells. It is associated with allergic diseases such as asthma and hay-fever.

In addition to the five main class or classes, there are four subclasses for IgG (IgG1, IgG2, IgG3 and IgG4). Additionally there are two subclasses for IgA (IgA1 and IgA2).

There are two types of light chain: Lambda (λ) and Kappa (κ). There are approximately twice as many κ as λ molecules produced in humans, but this is quite different in some mammals. Each chain contains approximately 220 amino acids in a single polypeptide chain that is folded into one constant and one variable domain. Plasma cells produce one of the five heavy chain types together with either κ or λ molecules. There is normally approximately 40% excess free light chain production over heavy chain synthesis. Where the light chain molecules are not bound to heavy chain molecules, they are known as "free light chain molecules". The κ light chains are usually found as monomers. The λ light chains tend to form dimers.

The heavy chains and light chains of antibodies are normally attached together via disulphide bonds. These bonds are relatively weak and can be broken to release the heavy chain and light chains from each other.

This is a problem where antibodies might be used on conditions where the disulphide bond might be expected to break, such as under reducing conditions. This would allow the antibody to dissociate and reduce the binding activity of the antibody.

The Applicant has realised that there are circumstances where it would be advantageous to be able to use antibodies under reducing conditions to allow the analysis of partially or full denatured proteins.

For example, there are a number of clinically important diseases which are characterised by splice variants or polymorphisms. Often such variants are normally hidden within the folding of the protein. Reducing conditions may be used to open the protein structure and allow the detection of such variants.

Splice variants are known to be present in a variety of cancer markers, for example. They are often currently found at the nucleic acid level using, for example real-time PCR. This is reviewed by, for example, Chem. J. and Weiss WA, Oncogene (2015) 34, 1-4. It is expected that next generation precision medicine therapies may target specific splice variants or isoforms. The ability to detect those variations using rapid, point of care test kits, even where variations are usually masked by protein folding is clearly advantageous.

Other areas where reducing conditions light be used to identify changes, includes the study of phosphorylation by kinases. The sites of phosphorylation are often hidden by protein folding.

Albumins or other carrier proteins in the body, are often bound to molecules of interest, such as hormones. Albumin, for example, is the most abundant blood protein. It acts as a carrier to non-specifically bind several hydrophobic steroid hormones, hemin and fatty acids. There may be release and detected immunologically under conditions that might affect the structure of the antibodies used.

Other carrier proteins are found in cells and are used to transport molecules across cell membranes. Indeed defects in specific carrier proteins have been associated with a number of diseases.

Alternative systems where harsh or reducing conditions might be used, is in the study of urinary micelles and serum exosomes. These often contain compounds of clinical interest which need detecting. Exosomes, for example, are small membrane bound residues secreted by most cell types. They contain various functional proteins, mRNAs and mirRNAs of diagnostic and therapeutic interest (Rekker et al Clin. Biochem. (2014) 135-138).

The ability to immunoassay for different epitopes under conditions where the antibody would normally be expected to dissociate is therefore advantageous.

WO2006/099481A describes the use of intra- and interchain thioether cross links in a wide range of macromolecules including polypeptides such as polyclonal antibodies, monoclonal antibodies, Fab, F(ab) and F(ab')₂ fragments, single chain antibodies, human antibodies, harmonised or chimeric antibodies and epitope binding fragments. The document describes that the aim of the cross-linking is to enhance the stability and pharmaceutical and functional properties of the antibody or fragment. In particular, the aim is to cross-link, for example, the heavy and light chains of different monoclonal antibodies, such as anti-viral antigen antibodies, including anti-RSV antibodies. The stated aim is to improve the pharmaceutical properties of the antibodies.

WO00/44788 describes using thioethers to cross-link different antibody molecules of different specificities with the aim of producing improved therapeutic agents. Similarly, bi- or tri-specific (Fab)₃ or (Fab)₄ conjugates with different specificities is shown in WO91/03493.

Thioethers have been observed in therapeutic antibodies with increasing levels on storage (Zhang Q *et al* JBC manuscript (2013) M113.468367). A light chain-heavy chain disulphide (LC214-HC220) can convert to a thioether bond. One IgGlk therapeutic antibody was observed to convert to a thioether at that position at a rate of 0.1% per day whilst circulating in blood. Endogenous antibodies were also observed to be formed in healthy human subjects. Zhang *et al* repeated the thioether formation *in vitro.* This was used to help assess the safety impact of the thioether bonds on therapeutic monoclonal antibodies. Goldberg et al., Bioconjugate Chem. 1991, 2, 275 discloses bismaleimide-linked antibodies, their use in ELISA, and their stability to reducing and denaturing agents.

KR 2009/0094822 discloses the use of antibodies for detection of a target under denaturing conditions in a Western blot.

In contrast to Zhang which considered the formation of cross-links to be problematic, the Applicant has realised that for some uses the formation of cross-linked antibodies can have unexpected advantages.

The invention is set out in the appended claims. In particular, the invention covers a method for analysis of partially or full denatured proteins by detecting an analyte in a sample using an immunoassay kit, comprising adding a reducing agent from the immunoassay kit to the sample to detect the presence of, or an amount of, the analyte in the sample and detecting the analyte with one or more analyte specific antibodies or fragments thereof from the immunoassay kit, the one or more analyte specific antibodies or fragments thereof comprising one or more non-disulphide cross-links between at least one heavy chain or fragment thereof and at least one light chain or fragment thereof of the analyte-specific antibodies or fragments thereof characterised that the one or more analyte specific antibodies or fragments detect the presence of, or the amount of, the analyte in the sample under reducing conditions.

Cross-links between adjacent heavy chains may also be provided in addition to the heavy chain - light chain cross-links.

The immunoassay may be any immunoassay in which the presence of a specific antigen or often called the analyte, is determined by the specific binding of the antibody or fragment to the antigen.

The immunoassay is carried out on a sample. The sample may have been pre-treated to remove contaminants or concentrate the analyte of interest. This may include treating the sample with a reducing agent to denature the sample before or after adding the antibodies or fragments.

Reducing agents include B-mercaptoethanol and dithiothreitol.

The cross-links are typically intramolecular between chains of the same antibody

The cross-link typically comprises a thioether bond.

A thioether cross-link comprises a thioether bond. This is a link between residues of the antibody wherein the link has a single sulphur bond rather than a disulphide bond. That is thioether cross-links do not include links that comprise more than one sulphur atom, such as disulphide bridges that are familiar to those skilled in the art. Instead, a thioether cross-link comprises a single sulphur bond that bridges residues of a macromolecule. One or more additional non-sulphur atoms may additionally form the link.

The residues linked by thioether cross-links can be natural residues or non-natural residues. Formation of the thioether cross-link can result in a loss of atoms from the residues, as will be recognised by those skilled in the art. For example, formation of a thioether cross-link between side chains of two cysteine residues can result in loss of a sulphur atom and hydrogen atoms from the residues, yet the resulting thioether cross-link will be recognised as linking the cysteine residues by one skilled in the art.

Thioether cross-links can link any two residues of the antibody. One or more of the residues may be selected, for example, from cysteine, aspartic acid, glutamic acid, histidine methionine and tyrosine. Two of the residues may be selected from the group consisting of cysteine, aspartic acid, glutamic acid, histidine, methionine and tyrosine. More typically two of the residues are cysteine residues. Typically, only one thioether cross-link is between the heavy chain and the light chain. Alternatively, two, three or more thioether cross-links may be used. The heavy chain pair of the antibody, or a fragment thereof, may also be linked by one or more non-disulphide cross-links, such as thioether bonds.

Thioether cross-links are described in, for example, WO2006/099481, and Zhang et al (2013) J. Biol. Chem. vol 288(23), 16371-8 and Zhang & Flynn (2013) J. Biol. Chem, vol 288(43), 34325-35.

Phosphines and phosphites may be used. Here, 'Phosphine' refers to any compound containing at least one functional unit with the general formulae R₃P (where P = phosphorous and R = any other atom). In phosphites, the R positions are occupied specifically by oxygen atoms. R₃P-containing compounds act as strong nucleophiles that can attack disulphide bonds. This can result in reduction of disulphides, however under some conditions, may also result in thioether bond formation.

Compounds include:
Tris(dimethylamino)phosphine (CAS Number 1608-26-0)
Tris(diethylamino)phosphine (CAS Number 2283-11-6)
Trimethylphosphite (CAS Number 121-45-9)
Tributylphosphine (CAS Number 998-40-3)
References: Bernardes et al. (2008) Angew. Chem. Int. Ed., vol 47, 2244-2247

Cross-links may also comprise cross-linkers such as a maleimido cross-linker, which reacts with three thiols to cross-link to chains of the antibody molecule. This can be made to bind on one side of a thiol group and additionally on another moiety such as a lysine carboxyl group, as described in WO00/44788.

Bi-functional cross-linkers may be used comprising two reactive moieties linked together by a linker, especially a flexible linker. The linker may comprise one or more carbons covalently bound together in a chain, for example a substituted or non-substituted alkyl. The linker especially a C1-C10, most typically a C2-C6 or C3-C6 linker. The Applicants have found that C2-C6 containing cross linkers, such as, α,α'-Dibromo-m-xylene, BMOE (bismaleiimidoethane) or BMB (bismaleimidobutane) particularly useful with relatively high levels of recovery of cross-linked protein.

### Bismaleimide is a homobifunctional sulfhydryl reactive crosslinker

This is a well characterised class of cross-linker contains two maleimide groups connected by a hydrocarbon or other linker. The maleimide groups spontaneously react with free sulfhydryl groups exposed by reduction of disulphides to form a non-reducible thioether bond at each sulfhydryl, thereby covalently crosslinking the two remaining cystines.

Compounds include:
Bis(maleimido)ethane (CAS Number 5132-30-9)
1,4-bis(maleimido)butane (CAS Number 28537-70-4)

### References:

Auclair et al. (2010) Strategies for stabilizing superoxide dismutase (SOD1), the protein destabilized in the most common form of familial amyotrophic lateral sclerosis. Proc Natl Acad Sci U S A, vol 107(50) - pages 21394-9 incorporated herein by reference
Geula at al. (2012) Structure-based analysis of VDAC1 protein: defining oligomer contact sites. J Biol Chem, vol 287(3), 475-85 incorporated herein by reference
Kida et al. (2007) Two translocating hydrophilic segments of a nascent chain span the ER membrane during multispanning protein topogenesis. J Cell Biol, vol 171(7) pages 1441-1452 incorporated herein by reference

### α,α'-Dibromo-m-xylene is a homobifunctional sulfhydryl reactive crosslinker which may also be used

Dibromo-*m*-xylene (CAS Number 626-15-3) is a member of the di-alkyl halide class of compounds and acts as a homobifunctional crosslinker that reacts with free sulfhydryl groups.

### Reference:

Jo et al. (2012) Development of α-Helical Calpain Probes by Mimicking a Natural Protein-Protein Interaction J Am Chem Soc., col 134(42) - pages 17704-13.

### Alternative sulfhydryl reactive cross-linking compounds forming stable thioether bonds

There are at least six classes of reagent known to react with free sulfhydryls and result in a non-reducible covalently cross-linked product. The specific reactivity of these compounds to sulfhydryl groups varies and some will react with water, amines and carboxyl groups under certain conditions. In addition, many of these compounds have bulky linker groups, which may limit their ability to cross-link in restricted spatial environments. The list below gives a few examples from each class, but a more comprehensive list and references is given in:
Chemistry of Protein Conjugation and Cross-linking, Wong, S: ISBN 0-8493-5886-8.

### Bismaleimides

bis(maleimido)hexane; N-N'-Methylenebismaleimide; Bis(N-maleimidomethyl)ether; N,N'-(1,3-Phenylene)-bismaleimide; Bis(N-maleimido)-4,4'-bibenzyl; Naphthalene-1,5-dimaleimide

### Haloacetyl derivatives

1,3-Dibromoacetone; N,N'-Bis(iodoacetyl)polmethylenediamine; N,N'-Di(bromoacetyl)phenylhydrazine; 1,2-Di(bromoacetyl)amino-3-pheylhydrazine; γ-(2,4-Dinitrophenyl)-α-bromoacetyl-L-diaminobutyric acid bromoacetyl-hydrazide

### Di-alkyl halides

α,α'-Dibromo-*p*-xylene sulfonic acid; α,α'-Diiodo-*p*-xylene sulfonic acid; Di(2-chloroethyl)sulphide; Tri(2-chloroethyl)amine; N,N-Bis(β-bromoethyl)benzylamine 2.4 s-Triazines

Dichloro-6-methoxy-s-triazine; 2,4,6-Trichloro-s-triazine (Cyanuric acid); 2,4-Dichloro-6-(3' -methyl-4-aminoanilino)-s-triazine; 2,4-Dichloro-6-amino-s-triazine

### Aziridines

2,4,6-Tri(ethyleneimido)-s-triazine; N,N' -Ethyleneiminoyl-1,6-diaminohexane; Tri[1-(2-methylaziridenyl)]-phosphine oxide

### Bis-epoxides

1,2:3,4-Diepoxybutane; 1,2:5,6-Diepoxyhexane; Bis(2,-epoxypropyl)ether; 1,4-Butadioldiglycidoxyether

The cross-link may replace one or more naturally occurring disulphide bonds or alternatively may be produced in addition to the disulphide bond.

Typically at least 50%, at least 60 %, at least 70 %, at least 80%, at least 90 % or at least 95% of the antibodies are cross-linked. Cross linking efficiencies of 70% - 80% have been observed using, for example, bismaleimide. The cross-linked antibodies may be further purified to produce higher levels of cross-linking, for example by adding a reducing agent to break the disulphide bonds of remaining non-cross-linked antibodies and separating using, for example, gel electrophoresis.

The antibody or fragment may be anti-free light chain specific (such as anti-kappa free light chain or anti-lambda free light chain), anti-heavy chain subclass specific, anti heavy chain type specific, anti-heavy chain class-light chain type specific.

Heavy chain classes may be IgG, IgA, IgM, IgD or IgE, typically IgG or IgA.

Heavy chain subclasses include IgA1, IgA2, IgG1, IgG2, IgG3 and IgG4.

The antibodies may be species specific, such as anti-human or anti-horse or anti-sheep or anti-pig. The antibody may be raised in a cartilaginous fish, a camelid such as llama, cow, rabbit, rat, sheep, goat, horse or mouse.

The antibodies or fragments are capable of specifically binding immunoglobulins.

Typically the antibody or fragment is attached to a support. This may be, for example, any suitable chromatographic support generally known in the art for protein purification, such as an immuno-purification of antibodies or fragments. These include: magnetic beads, agarose resins and other supports generally known in the art. The covalent and non-covalent attachment of antibodies to supports is generally known in the art. This may be achieved, for example by reacting free amine groups on the antibodies with a support activated with and agent such as cyanogen bromide, N-hydroxysuccinimide (sulfo-NHS) or tresyl chloride. The water soluble carbodiimide , EDC, may be used to form active ester functionalities with carboxylate groups using sulfo-NHS. The use of biotinylated antibodies or supports to bind to the counterpart streptavidin moieties on the other of the support or antibodies has also been used in the art.

The antibody or fragment thereof may be a monoclonal antibody. Alternatively, the antibody or fragment may be a polyclonal antibody or fragment.

A mixture of antibodies, or fragments, having different specificities may be provided.

The fragment of the antibody may, for example, be an Fab or F(ab')₂ fragment.

The sample is typically a sample of serum from a subject. However, whole blood, plasma, urine or other samples of tissue or fluids may also potentially be utilised. Alternatively, the sample may be an environmental sample or a sample from an animal, plant, bacterium or fungus.

The assay may be an ELISA type assay or utilise fluorescent beads such as Luminex^{™} beads.

Sandwich assays, for example use antibodies to detect specific antigens. One or more of the antibodies used in the assay may be labelled with an enzyme capable of converting a substrate into a detectable analyte. Such enzymes include horseradish peroxidase, alkaline phosphatase and other enzymes known in the art. Alternatively, other detectable tags or labels may be used instead of, or together with, the enzymes. These include radioisotopes, a wide range of coloured and fluorescent labels known in the art, including fluorescein, Alexa fluor, Oregon Green, BODIPY, rhodamine red, Cascade Blue, Marina Blue, Pacific Blue, Cascade Yellow, gold; and conjugates such as biotin (available from, for example, Invitrogen Ltd, United Kingdom). Dye sols, chemiluminescent labels, metallic sols or coloured latex may also be used. One or more of these labels may be used in the ELISA assays described herein or alternatively in the other assays, labelled antibodies or kits described herein.

The construction of sandwich-type assays is itself well known in the art. For example, a "capture antibody" specific for the analyte is immobilised on a substrate. The "capture antibody" may be immobilised onto the substrate by methods which are well known in the art. FLC in the sample are bound by the "capture antibody" which binds the FLC to the substrate via the "capture antibody".

Unbound immunoglobulins may be washed away.

In ELISA or sandwich assays the presence of bound immunoglobulins may be determined by using a labeled "detecting antibody" specific to a different part of the analyte of interest than the binding antibody.

Competition assays in which the sample analyte competes for binding to the antibodies with a predetermined amount of analyte, may also be used. Typically the predetermined analyte is labelled with a detectable label, such as those discussed above.

Flow cytometry may be used to detect the binding of the analyte of interest. This technique is well known in the art for, e.g. cell sorting. However, it can also be used to detect labeled particles, such as beads, and to measure their size. Numerous text books describe flow cytometry, such as Practical Flow Cytometry, 3rd Ed. (1994), H. Shapiro, Alan R. Liss, New York, and Flow Cytometry, First Principles (2nd Ed.) 2001, A.L. Given, Wiley Liss incorporated herein by reference.

One of the binding antibodies, such as the antibody specific for analyte, is bound to a bead, such as a polystyrene or latex bead. The beads are mixed with the sample and the second detecting antibody. The detecting antibody is preferably labeled with a detectable label, which binds the analyte to be detected in the sample. This results in a labeled bead when the analyte to be assayed is present.

Other antibodies specific for other analytes described herein may also be used to allow the detection of those analytes.

Labeled beads may then be detected via flow cytometry. Different labels, such as different fluorescent labels may be used for, for example, different anti-analyte antibodies. This allows the amount of each type of analyte bound to be determined simultaneously or the presence of other analytes to be determined.

Alternatively, or additionally, different sized beads may be used for different antibodies, for example for different marker specific antibodies. Flow cytometry can distinguish between different sized beads and hence can rapidly determine the amount of each analyte or other analyte in a sample.

An alternative method uses the antibodies bound to, for example, fluorescently labeled beads such as commercially available Luminex^{™} beads. Different beads are used with different antibodies. Different beads are labeled with different fluorophore mixtures, thus allowing different analytes to be determined by the fluorescent wavelength. Luminex beads are available from Luminex Corporation, Austin, Texas, United States of America.

Preferably the assay used is a nephelometric or turbidimetric method. Nephelometric and turbidimetric assays for the detection of analytes are generally known in the art.

Preferably the method comprises detecting the amount of analyte in the sample utilising the immunoassay.

The antibodies may be polyclonal or monoclonal. Polyclonal antibodies may be used because they allow for some variability between light chains of the same type to be detected as they are raised against different parts of the same chain. The production of polyclonal antibodies is described, for example in WO97/17372.

Fragments of antibodies, such as (Fab')₂ or Fab antibodies, which are capable of binding FLC may also be used.

The antibodies or fragments may be labelled, for example with a label as described above. Labelled anti-immunoglobulin binding antibodies or fragments thereof may be provided to detect anti-analyte antibodies or fragments bound to the analyte.

The kit may comprise calibrator fluids to allow the assay to be calibrated. The kit may also be adapted by optimising the amount of antibody and "blocking" protein coated onto the latex particles and, for example, by optimising concentrations of supplementary reagents such as polyethylene glycol (PEG) concentrations. One or more reducing agents such as B-mercaptoethanol and dithiothreitol may also be provided.

The kit may comprise, for example, a plurality of standard controls for the analyte. The standard controls may be used to validate a standard curve for the concentrations of the analyte or other components to be produced. Such standard controls confirm that the previously calibrated standard curves are valid for the reagents and conditions being used. They are typically used at substantially the same time as the assays of samples from subjects.

Lateral flow test kits may be used. These are traditionally immunochromotographic assays. They are used to rapidly identify the presence or absence of a particular analyte in a sample. The assay is usually enclosed inside a plastic housing as a test strip, or dipstick, with specialised regions to receive, process and view the sample. The test strip is often fixed to an inert backing. Samples do not usually require pre-treatment. Lateral flow assays can also be integrated with electronic systems, for example, to indicate a quantity of the analyte in a readable format.

A lateral flow test strip usually comprises four separate regions; a sample pad, a conjugate or reagent pad, a reaction membrane and an adsorbent pad or waste reservoir.

The sample pad is usually made of cellulose and/or glass fibres. The sample pad receives the sample, which is typically a biological liquid sample such as urine. As the sample comes into contact with the sample pad, the assay is activated. The liquid sample migrates through the sample pad, moving laterally across the length of the test strip. The movement of the sample is intended to be homogenous and continuous. Sample pads may also contain reagents which are required for pre-treatment of the sample, including separation, buffering, filtering or cleaning.

The conjugate or reagent pad is where the conjugated ligands are located which have been selected for their ability to specifically bind to the analyte of interest. The ligands are conjugated to a label. They are immobilised by temporary adsorption to the conjugate pad by a bond which is broken upon contact with a liquid. The porosity, structure and composition of the material the conjugate pad is formed from can be selected for particular desired properties including sensitivity and release rates. Typically, conjugate pads are formed from cellulose, glass fibres and/or various polyesters. The labelled ligand binds to the analyte of interest in the liquid sample and is transported laterally onwards through the conjugate pad matrix. Typically, the ligands are polyclonal antibodies which bind specifically to the analyte of interest. Aptamers may also be used as ligands. The ligands can be labelled by a number of conjugate labels including enzymes, coloured dyes, fluorescent dyes or nanoparticles.

The reaction membrane is usually a nitrocellulose membrane. Membranes are graded and can be selected for a required sensitivity, binding properties and support. A test line and a control line are located on the reaction membrane. The liquid sample containing unbound analyte and analyte bound to the labelled ligand will be adsorbed onto the membrane. The liquid moves across the reaction membrane via capillary action.

The absorbent pad or reservoir acts as a sponge to adsorb excess liquid sample and regulates the continuous flow of the liquid across the test strip. The properties of the absorbent pad will determine the adsorption rate across the test strip.

There are two types of lateral flow assays; sandwich and competitive. Sandwich assays which use antibodies are most common. In a sandwich assay, the test line contains immobilised primary antibodies which are specific for the analyte of interest. Secondary antibodies which are specific for the conjugated labelled antibodies located in the conjugate pad are immobilised at the control line. The sample is applied to the sample pad where it travels to and contacts the conjugate pad. The analyte of interest in the liquid sample binds to the conjugated labelled antibody here and travels to the test line. At the test line, the primary antibody binds to the analyte which may or may not be bound to the conjugated labelled antibody. If the analyte is bound to the conjugated labelled antibody from the conjugate pad, the analyte is said to be "sandwiched" between the primary antibody and the conjugated labelled antibody. The liquid sample then contacts the control line, where the secondary antibodies bind any excess remaining conjugated labelled antibody. Any excess liquid sample will travel on to the absorbent pad. The increased number of labelled antibodies at the test line in close proximity to one another will result in a visible change (such as a colour change), whilst a visible change at the control line indicates that the test is working correctly. The intensity, for example, of the colour change at the test line may be interpreted to indicate a quantitative value of the analyte.

Competitive assays are often used for smaller molecules which are unable to bind two binding ligands, such as antibodies, simultaneously. The sample pad and conjugate pad function in the same way as in a sandwich assay, but the test line contains the analyte of interest which is immobilised to the test line. As the sample liquid reaches the test line, the immobilised analyte binds the conjugated labelled ligand. If the analyte is at low levels in the initial liquid sample, a larger number of the conjugated labelled ligands will bind to the test line. The analytes are said to compete for the binding site on the conjugated labelled ligand. A subsequent increase in, for example, colour intensity at the test line indicates the absence or low levels of the analyte in the liquid sample. This can also be measured quantitatively. A secondary ligand specific for the conjugated labelled ligand is immobilised to the control line to indicate that the test is working correctly.

Lateral flow tests usually have a very long shelf life and remain stable over a variety of environmental conditions. The sample volume required is relatively small, for example, a typical pregnancy test requires 25ml of urine or 5mls of blood. Lateral flow tests can also be multiplex. These tests can detect the presence of more than once analyte simultaneously.

Recently, lateral flow tests have been developed for use with metal nanoparticles, particularly gold nanoparticles. Gold nanoparticles are used to label ligands which bind to the analyte of interest. Typically, colloidal gold nanoparticles are used as they are relatively inert and can produce highly spherical particles. These particles are usually charged which ensures they have an affinity for protein molecules. Proteins can be adsorbed against or covalently attached to the surface of gold particles.

The assay kit may be a nephelometric or turbidimetric kit, an ELISA, flow cytometer, fluorescent, chemiluminescent, radioimmune, bead-type or lateral flow kit.

The invention will now be described by way of example only with respect to the following figures
**Figure 1** shows Coomassie Blue stained gels illustrating the formation of thioether bonds in anti-free kappa F(ab')₂ and anti-free lambda F(ab')₂ antibody fragments. The formation of cross-linked light and heavy chains of the F(ab')₂ molecule are indicated in the SDS-PAGE gel image (arrow at approximately 50kDa). The degree of cross-linking is higher at 7 days.
**Figure 2** shows Coomassie Blue stained gels of treated (to induce thioether bond formation) and non-treated anti-free kappa F(ab')₂ fragments. (NR) non-reduced samples (R) reduced samples. Interpretation of the structures in each band is also shown.
**Figure 3** similarly shows anti-free lambda F(ab')₂ antibody fragments.
**Figure 4** shows the binding activity of thioether cross-linked and non-cross-linked anti-free kappa and anti-free lambda F(ab')₂ antibodies to free light chains (FLC) and normal human serum (NHS).
**Figure 5** shows the cross-linking of anti-lambda total F(ab')₂ antibodies by bismaleimidoethane (BMOE), as measured by reducing Coomassie Blue stained SDS-PAGE.
**Figure 6** shows the binding of anti-lambda total F(ab')₂ antibodies to IgG lambda before and after bismaleimidoethane (BMOE) cross-linking.
**Figure 7** shows anti-free lambda F(ab')₂ (10 µM) cross-linked with 2 mM bismaleimidoethane (BMOE) and anti-free kappa whole molecule (10 µM) cross-linked with 4 mM BMOE, as measured by reducing Coomassie Blue stained SDS-PAGE. Scanning densitometry of the reduced SDS-PAGE lanes indicated a cross-linking efficiency of >70% for anti-free lambda and >90% for anti-free kappa.
**Figure 8** shows untreated and bismaleimidoethane (BMOE) cross-linked anti-free kappa whole molecule binding to kappa free light chains. Cross-reactivity to IgG, IgA and IgM is also shown.
**Figure 9** shows untreated and bismaleimidoethane (BMOE) cross-linked anti-free lambda F(ab')₂ binding to lambda free light chains. Cross-reactivity to IgG, IgA and IgM is also shown.

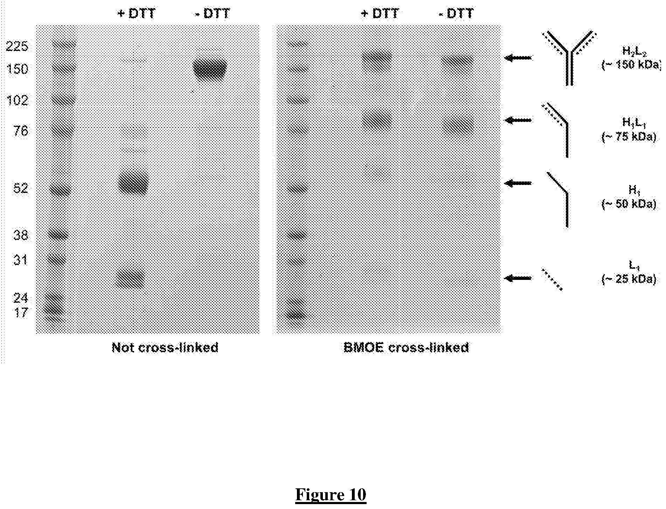

**Figure 10** shows the cross-linking and stabilisation of anti-prealbumin antibodies by bismaleimidoethane (BMOE) treatment, as illustrated by Coomassie Blue stained SDS-PAGE analysis. Without prior cross-linking (left panel), the antibodies spontaneously disassociated into their constituent heavy (H₁) and light (L₁) chain fragments in the presence of reducing agent (50 mM Dithiothreitol; + DTT). Following treatment with BMOE (right panel) the antibodies became resistant to reducing conditions and migrated at apparent molecular weights consistent with the formation of stable heavy-light chain pairs (H₁L₁ and H₂L₂). Interpretation of the structures in each band is shown.
**Figure 11** shows the binding activity of BMOE-stabilised anti-prealbumin antibodies (Xa.PA) by ELISA analysis. Anti-prealbumin antibodies were treated with a range of chemical conditions prior to the addition of purified prealbumin protein and bound prealbumin was detected with biotinylated anti-prealbumin using Streptavidin-HRP. Binding activity is shown relative to unstabilised anti-prealbumin (a.PA). (PBS), Phosphate Buffered Saline; (DTT), 50 mM Dithiotheitol; (Tween), 0.2% Tween-20; (SDS), 0.1% Sodium Dodecyl Sulfate.

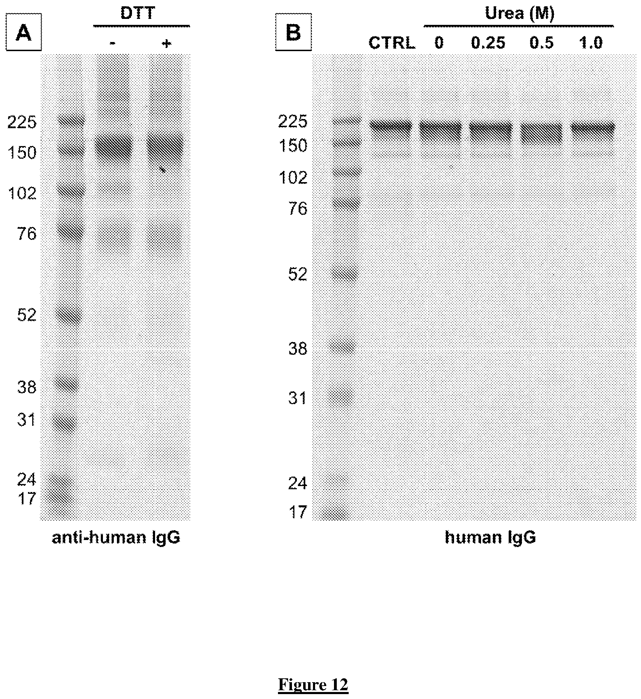

**Figure 12** shows the cross-linking of anti-IgG antibodies by BMOE (A) and the binding activity of BMOE-stabilised anti-IgG antibodies following chemical treatment (B), as illustrated by Coomassie Blue stained SDS-PAGE analysis. Panel (A) shows that BMOE-treated anti-IgG antibodies are resistant to 50 mM DTT. In panel (B), stabilised antibodies were immobilised on a solid matrix and treated with Phosphate Buffered Saline (CTRL) or a mixture of Glycine (0.1M, pH 3), tris(2-carboxyethyl)phosphine (TCEP, 2 mM) and 3-[(3-cholamidopropyl)dimethylammonio]-1-propanesulfonate (CHAPS, 0.3%) containing varying amounts of Urea. Following the addition of purified human IgG, specifically bound proteins were eluted with 0.1M Glycine pH 2.5 and analysed by SDS-PAGE.

The conversion of antibody disulphide bonds to thioether bonds may be induced in alkaline environments at raised temperature. Formation of such bonds is generally known in the art, such as in Zhang et al IgG1 Thioether Bond formation in vivo. JBC, 288:16371-16382, 2013. Zhang and Flynn. Cysteine racemization IgG heavy and light chains, JBC, 288:34325-34335, 2013.

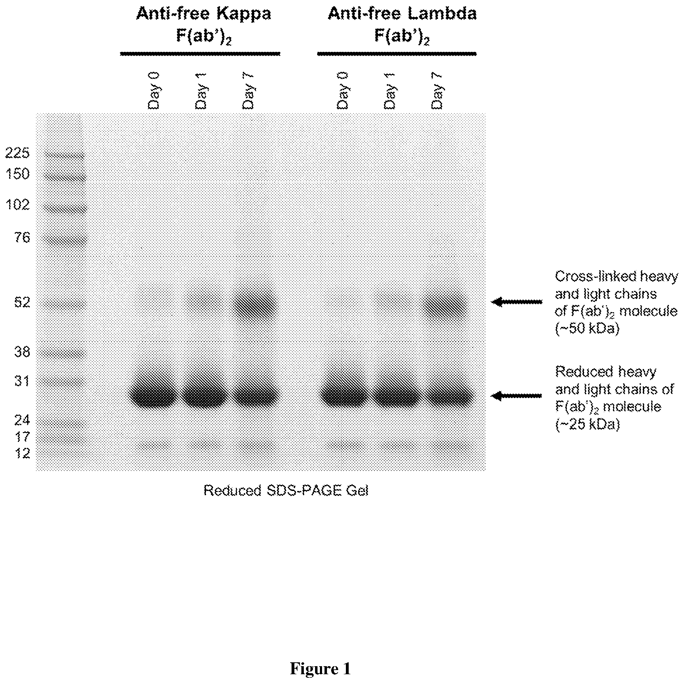

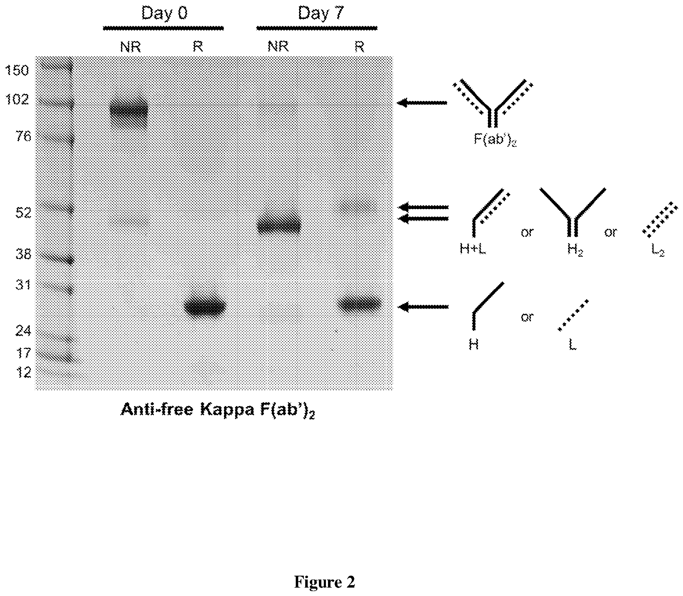

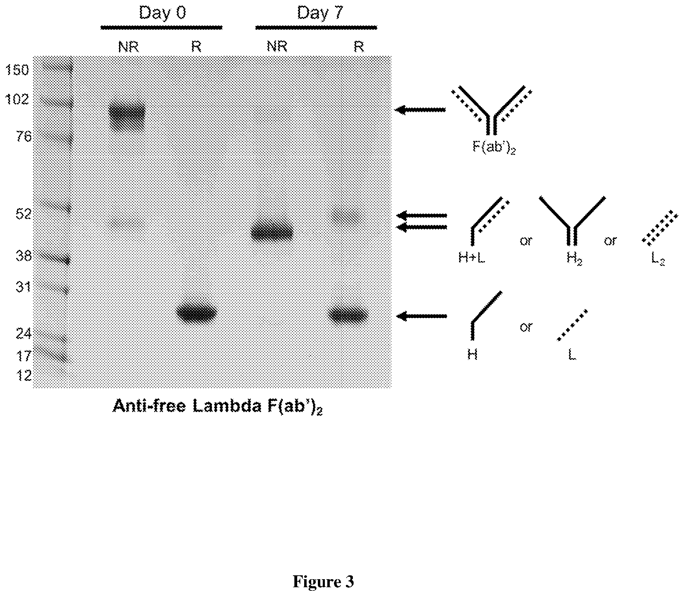

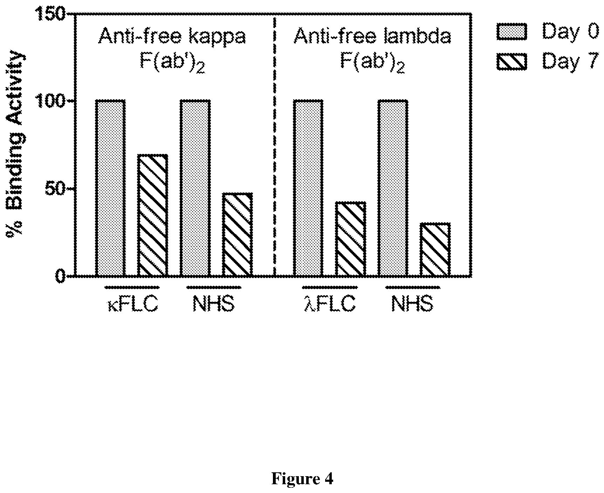

Anti-kappa free light chain and anti-lambda free light chain F(ab)₂ antibodies were investigated to see whether thioether bonds could be introduced into the fragments. The Applicant manufactures anti-kappa and anti-lambda antisera and sells immunoassays that utilise anti-kappa and anti-lambda antisera under the trade mark Freelite^{™.} These antibodies bind either free lambda or free kappa light chains. The data shown in Figures 1-4 shows that it is possible to introduce thioether cross-links into F(ab')2 fragments by treatment with 50 mM Glycine-NaOH, pH 9 at 50°C. Approximately 20% cross-linking efficiency is observed compared to untreated F(ab')2 after 7 days (Figures 1-3). Whilst there is some reduction in ELISA activity, as shown in Figure 4, activity still remains in the treated antibodies.

The binding activity of anti-free kappa and anti-free lambda antibodies was assessed by ELISA after 0 and 7 days alkaline treatment (Figure 4). Antibodies were coated onto ELISA plates and presented with purified kappa light chains, purified lambda light chains or Normal Human Serum. Binding activity was detected by measuring absorbance at 450nm using Tetramethylbenzidine (TMB) chromogenic substrate and anti-light chain antibodies conjugated to Horse Radish Peroxidase (HRP). Whilst there is some reduction in ELISA activity, as shown in Figure 4, activity still remains in the treated antibodies

### Bismaleimidoethane (BMOE) crosslinking of anti-lambda total F(ab')2 fragments

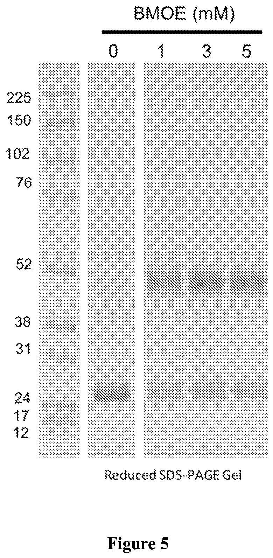

Anti-lambda F(ab')2 antibodies were investigated to see if antibody chains could be cross-linked by BMOE. Anti-lambda F(ab')2 fragments were reduced with 1 mM (TCEP). The TCEP was removed using Hi-Trap Desalting columns and the reduced anti-lambda total F(ab')₂ was cross-linked at 100-500x fold molar excess of BMOE and then analysed by Coomassie Blue stained SDS-PAGE run under reducing conditions. Figure 5 shows that BMOE can cross-link F(ab')2 fragments with an efficiency of over 50%. Moreover, the resulting antibody chain cross-links are resistant to reducing conditions.

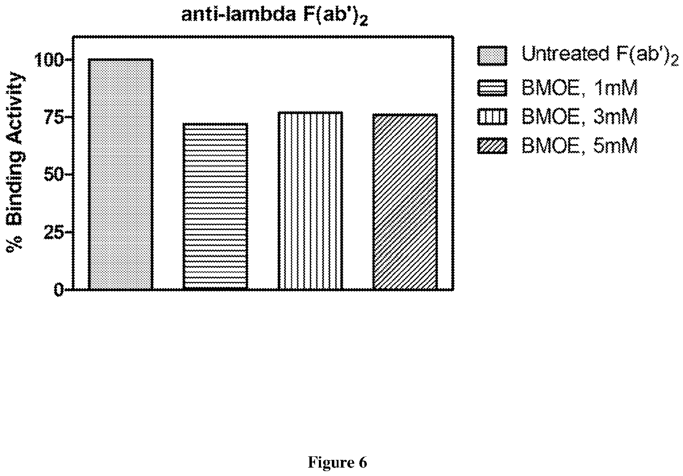

An ELISA plate was coated with polyclonal IgG Lambda and BMOE-treated or untreated anti-total lambda F(ab')₂ was bound to the plate. Binding activity by anti-total lambda was measured by light absorbance at 450nm using anti-sheep-HRP and TMB substrate. Under conditions that produce >50% BMOE cross-linking (Figure 5), anti-total lambda antibody retains over 70% IgG Lambda binding activity (Figure 6).

### BMOE cross-linked anti-free lambda and anti-free kappa antisera

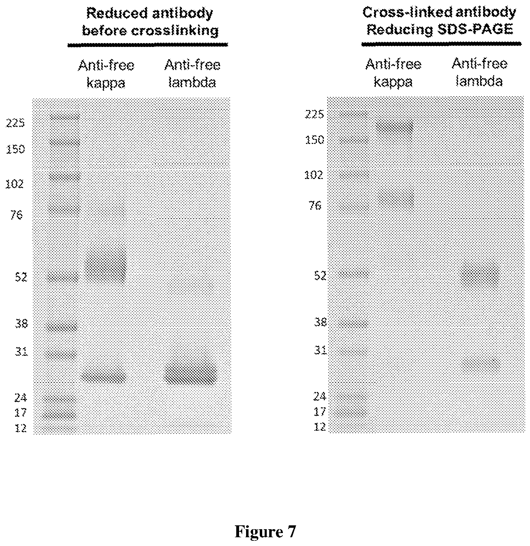

Commercially available Freelite^{™} antibodies were investigated to see whether antibody chains could be cross-linked by BMOE treatment. Whole molecule anti-free kappa and F(ab')₂ anti-free lambda were reduced with 1.5 mM and 1.0 mM TCEP, respectively. The TCEP was removed using Hi-Trap Desalting columns and the anti-free kappa and anti-free lambda antibodies were cross-linked at 400-fold and 200-fold molar excess of BMOE, respectively. Samples were analysed by Coomassie Blue stained SDS-PAGE run under reducing conditions. As shown in Figure 7, the BMOE cross-linking efficiency is over 50% for antibodies in either F(ab')2 or whole molecule formats.

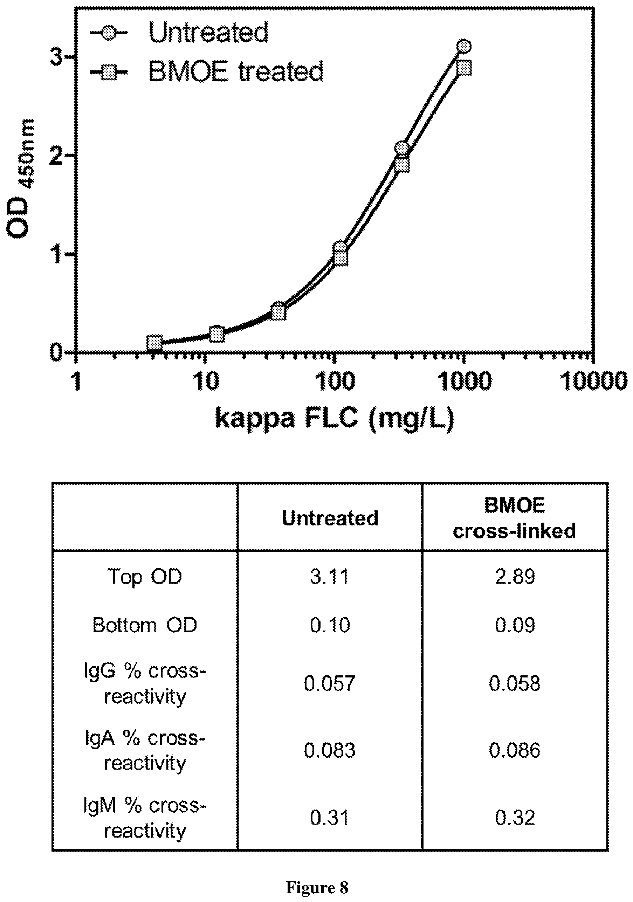

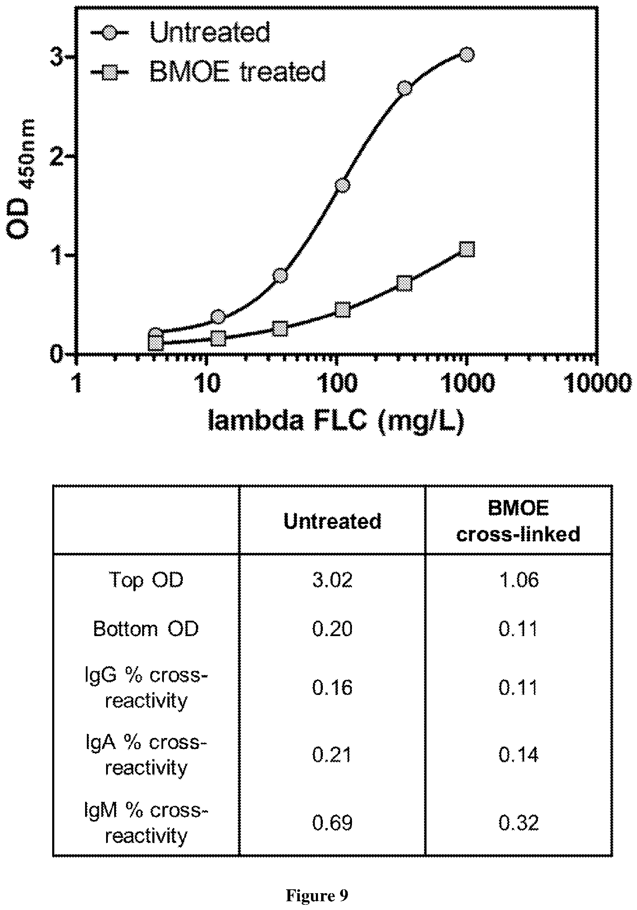

Activity ELISA assays were performed whereby BMOE-treated and untreated antibodies were coated onto ELISA plates and presented with purified immunoglobulins (IgG, IgA, IgM, free kappa, free lambda). Binding activity was detected by measuring absorbance at 450nm using anti-light chain-HRP and TMB substrate. The results in Figures 8-9 show that cross-linked antisera still maintain specific antigen binding activity.

### Stabilisation of anti-prealbumin antibodies by treatment with BMOE.

Anti-human prealbumin antibodies were investigated to see if antibody chains could be cross-linked by BMOE. Anti-prealbumin fragments were reduced with 250-fold molar excess of tris(2-carboxyethyl)phosphine (TCEP). The TCEP was removed using Hi-Trap Desalting columns and the reduced anti-prealbumin was cross-linked at 400-fold molar excess of BMOE and then analysed by Coomassie Blue stained SDS-PAGE run in the presence or absence of reducing agent (50 mM DTT). Figure 10 shows that without prior cross-linking, the antibodies spontaneously break down into their constituent heavy and light chains upon DTT treatment. In contrast, greater than 80% of the BMOE-treated antibodies remained associated as heavy-light chain pairs (in H₂L₂ or H₁L₁ forms) irrespective of the presence of DTT, thus indicating a high degree of structural stability.

### The antigen binding activity of BMOE-stabilised anti-prealbumin is resistant to reducing and detergent conditions.

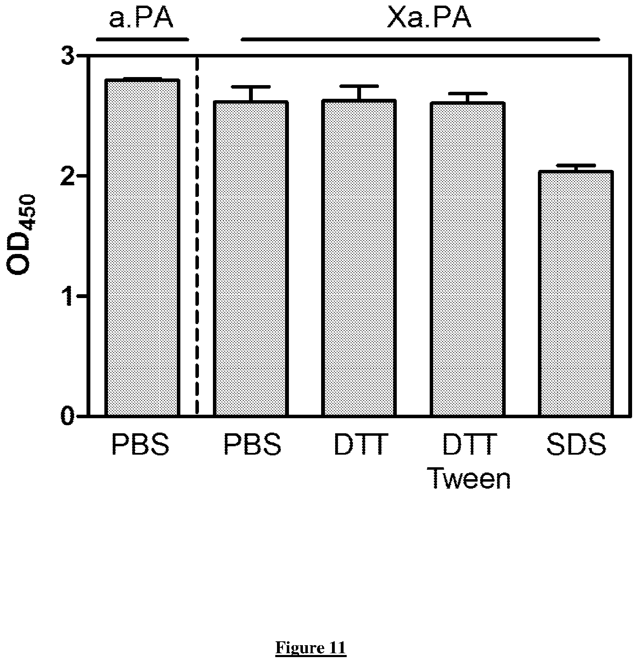

Conventional mammalian antibodies (e.g. IgG) express an antigen binding site (also known as the Complementarity Determining Region or Paratope) on each F(ab) fragment. These sites are formed by the associated variable domains from paired heavy and light chains, which both contribute to antigen recognition and binding activity. Therefore, under conditions that disrupt heavy-light chain pairing, the binding activity of antibodies for their target antigen would be compromised. As BMOE-treatment was shown in Figure 10 to stabilise heavy-light chain pairing, the binding activity of anti-prealbumin was investigated to determine whether BMOE-stabilisation protects the antibodies from reducing conditions and protein denaturants. In Figure 11, anti-prealbumin antibodies (5 µg/mL) were coated onto microwell plates and treated with a range of chemical conditions prior to the addition of purified prealbumin protein (0.1 µg/mL). Bound prealbumin was detected with biotinylated anti-prealbumin (0.2 µg/mL) using Streptavidin-HRP and 3,3',5,5'-Tetramethylbenzidine chromogenic substrate (TMB). The data in Figure 11 show that BMOE cross-linking of anti-prealbumin (Xa.PA, PBS) only had a marginal effect on prealbumin binding activity when compared to unstabilised anti-prealbumin (a.PA, PBS). Furthermore, treatment of BMOE-stabilised anti-prealbumin with 50 mM DTT had no significant effect on activity, even in the presence of non-ionic detergent (Tween 20, 0.2%). It was also observed that BMOE-stabilised anti-prealbumin retained antigen binding activity after exposure to Sodium Dodecyl Sulfate (SDS, 0.1%), an anionic detergent generally known in the art to act as a protein denaturant. Therefore, the data in Figures 10 and 11 illustrate that cross-linking of anti-prealbumin antibodies with BMOE confers a high degree of structural stability, minimally affects antigen binding activity under physiological conditions and protects the antigen binding activity from reducing agents and protein denaturants.

### Anti-IgG antibodies are stabilised by BMOE cross-linking

Anti-human IgG antibodies were investigated to see if antibody chains could be cross-linked by BMOE. Anti-IgG fragments were reduced with 250-fold molar excess of tris(2-carboxyethyl)phosphine (TCEP). The TCEP was removed using Hi-Trap Desalting columns and the reduced anti-prealbumin was cross-linked at 400-fold molar excess of BMOE and then analysed by Coomassie Blue stained SDS-PAGE run in the presence or absence of reducing agent (50 mM DTT). The results in Figure 12A show that greater than 80% of the BMOE-treated antibodies remained associated as heavy-light chain pairs irrespective of the presence of DTT, thus indicating a high degree of structural stability.

The BMOE-stabilised anti-IgG antibodies were also investigated to determine what effect exposure to a combination of low pH, reducing agent, detergent and chaotropic agent would have on the antigen binding activity. Antibodies were cross-linked with BMOE and covalently attached to a solid matrix by conventional amine-linkage chemistry. The antibodies were subsequently treated with Phosphate Buffered Saline as a control (CTRL) or Glycine (pH 3 buffer), TCEP (reducing agent) and CHAPS (detergent) containing varying concentrations of Urea (Chaotrope). Purified human IgG was applied and the specifically captured proteins were eluted using Glycine pH 2.5 buffer and analysed by SDS-PAGE. As shown in Figure 12, the BMOE-stabilised anti-IgG antibodies retained antigen binding activity under all of the conditions tested. This illustrates that BMOE-stabilisation renders anti-IgG antibodies resistant to a range of chemical conditions.

## Claims

1. A method for analysis of partially or full denatured proteins by detecting an analyte in a sample using an immunoassay kit, comprising adding a reducing agent from the immunoassay kit to the sample to detect the presence of, or an amount of, the analyte in the sample and detecting the analyte with one or more analyte specific antibodies or fragments thereof from the immunoassay kit, the one or more analyte specific antibodies or fragments thereof comprising one or more non-disulphide cross-links between at least one heavy chain or fragment thereof and at least one light chain or fragment thereof of the analyte-specific antibodies or fragments thereof characterised that the one or more analyte specific antibodies or fragments detect the presence of, or the amount of, the analyte in the sample under reducing conditions.

2. A method according to claim 1, wherein the immunoassay is a radioimmune assay comprising radioisotopes, a lateral flow assay test strip or dipstick, an ELISA-type assay comprising an enzyme capable of converting a substrate into a detectable label, a nephelometric assay a turbidimetric assay, a flow cytometry assay comprising detectable particles, a fluorescent assay comprising fluorescent labels, chemiluminescent assay comprising chemiluminescent labels or bead-type assay comprising fluorescently labelled beads.

3. A method according to claims 1 or 2, wherein the cross-link comprises a bismaleimide or a thioether bond.

4. A method according to claims 1 to 3, wherein the one or more analyte specific antibodies or fragments thereof are attached to a support.

5. A method according to claims 1 to 4, wherein the antibody or fragment is a monoclonal antibody or fragment or a polyclonal antibody or fragment thereof.

6. A method according to claims 1 to 5, wherein the antibody is a fragment of an antibody and is an F(ab')₂ fragment.

7. A method according to any preceding claim comprising the use of one or more additional anti-immunoglobulin class or immunoglobulin-type specific antibodies ,or fragments thereof, or anti-free light class specific antibodies, or fragments therof.

## Patentansprüche

1. Verfahren zur Analyse von teilweise oder vollständig denaturierten Proteinen durch Nachweis des Analyten in einer Probe unter Verwendung eines Immunassay-Kits, umfassend die Zugabe eines Reduktionsmittels aus dem Immunassay-Kit zu der Probe, um das Vorhandensein oder die Menge des Analyten in der Probe festzustellen, und den Nachweis des Analyten mit einem oder mehreren analytspezifischen Antikörpern oder Fragmenten davon aus dem Immunassay-Kit, wobei der eine oder die mehreren analytspezifischen Antikörper oder Fragmente davon eine oder mehrere Nicht-Disulfid-Querverbindungen zwischen mindestens einer Schwerkette oder einem Fragment davon und mindestens einer Leichtkette oder einem Fragment davon der analytspezifischen Antikörper oder Fragmente davon umfassen, **dadurch gekennzeichnet, dass** der eine oder die mehreren analytspezifischen Antikörper oder Fragmente die Anwesenheit oder die Menge des Analyten in der Probe unter reduzierenden Bedingungen feststellen.

2. Verfahren nach Anspruch 1, wobei der Immunassay ein Radioimmunassay, der Radioisotope enthält, ein Lateral-Flow-Assay mit Teststreifen oder Teststäbchen, ein Assay vom ELISA-Typ, der ein Enzym enthält, das in der Lage ist, ein Substrat in eine nachweisbare Markierung umzuwandeln, ein nephelometrischer Assay, ein turbidimetrischer Assay, ein Durchflusszytometrie-Assay, der nachweisbare Partikel enthält, ein Fluoreszenz-Assay, der fluoreszierende Markierungen enthält, ein Chemilumineszenz-Assay mit Chemilumineszenz-Markierungen oder Bead-Typ-Assay mit fluoreszenzmarkierten Beads ist.

3. Verfahren nach Anspruch 1 oder 2, wobei die Vernetzung eine Bismaleimid- oder eine Thioetherbindung umfasst.

4. Verfahren nach einem der Ansprüche 1 bis 3, wobei der eine oder die mehreren analytspezifischen Antikörper oder Fragmente davon an einen Träger gebunden sind.

5. Verfahren nach einem der Ansprüche 1 bis 4, wobei der Antikörper oder das Fragment ein monoklonaler Antikörper oder Fragment oder ein polyklonaler Antikörper oder Fragment davon ist.

6. Verfahren nach einem der Ansprüche 1 bis 5, wobei der Antikörper ein Fragment eines Antikörpers, und zwar ein F(ab')2-Fragment, ist.

7. Verfahren nach einem der vorangehenden Ansprüche, umfassend die Verwendung von einem oder mehreren zusätzlichen gegen eine Immunglobulin-Klasse oder einen Immunglobulin-Typ spezifischen Antikörpern oder Fragmenten davon, oder von gegen eine freie Leichtkettenklasse spezifischen Antikörpern oder Fragmenten davon.

## Revendications

1. Procédé d'analyse de protéines partiellement ou totalement dénaturées par détection d'un analyte dans un échantillon en utilisant un kit de dosage immunologique,
consistant à :
- additionner un agent réducteur du kit de dosage immunologique à l'échantillon,
- détecter la présence, ou d'une quantité de l'analyte dans l'échantillon, et
- détecter l'analyte avec un ou plusieurs anticorps spécifiques de l'analyte ou fragments de ceux-ci du kit de dosage immunologique,
* le ou les anticorps ou fragments d'anticorps spécifiques de l'analyte comprenant une ou plusieurs liaisons transversales non-disulfure entre au moins une chaîne lourde ou un fragment de celle-ci et au moins une chaîne légère ou un fragment de celle-ci des anticorps ou fragments d'anticorps spécifiques de l'analyte,
procédé **caractérisé en ce que**
le ou les anticorps ou fragments d'anticorps spécifiques de l'analyte détectent la présence, ou la quantité de l'analyte dans l'échantillon dans des conditions réductrices.

2. Procédé selon la revendication 1,
dans lequel
l'essai immunologique est un essai radio-immunologique comprenant des radio-isotopes, une bandelette de test ou un bâtonnet de test à écoulement latéral, un essai de type ELISA comprenant une enzyme capable de convertir un substrat en un marqueur détectable, un test né-phélométrique, un test turbidimétrique, un test de cytométrie de flux comprenant des particules détectables, un test fluorescent comprenant des marqueurs fluorescents, un test chimioluminescent comprenant des marqueurs chimioluminescents ou un test de type bille comprenant des billes marquées par fluorescence.

3. Procédé selon les revendications 1 ou 2,
selon lequel la réticulation comprend un bismaléimide ou une liaison thioéther.

4. Procédé selon les revendications 1 à 3,
selon lequel le ou les anticorps spécifiques d'un analyte ou leurs fragments sont fixés sur un support.

5. Procédé selon les revendications 1 à 4,
selon lequel l'anticorps ou le fragment est un anticorps ou un fragment monoclonal ou un anticorps ou un fragment polyclonal de celui-ci.

6. Procédé selon les revendications 1 à 5,
selon lequel l'anticorps est un fragment d'anticorps et un fragment F(ab')2.

7. Procédé selon l'une quelconque des revendications précédentes, consistant à :
utiliser un ou plusieurs anticorps supplémentaires spécifiques de la classe des immunoglobulines ou du type d'immunoglobuline, ou de fragments de ceux-ci, ou d'anticorps spécifiques de la classe anti-lumière libre, ou de fragments de ceux-ci.
